# EUROPÄISCHE PATENTSCHRIFT

(11) **EP 3 747 341 B1**
(45) Veröffentlichungstag und Bekanntmachung des Hinweises auf die Patenterteilung: **19.07.2023**
(21) Anmeldenummer: 20174962.9
(22) Anmeldetag: 15.05.2020
(51) Int. Cl.: A61B 1/00, G02B 6/04, A61B 1/005, A61B 1/07, G02B 23/24

(54) **ENDOSKOP MIT VERKLEBTEM LICHTLEITER**
ENDOSCOPE WITH BONDED LIGHT GUIDE
ENDOSCOPE POURVU DE CONDUCTEUR DE LUMIÈRE COLLÉ

(30) Priorität: 03.06.2019 DE 102019114763
(43) Veröffentlichungstag der Anmeldung: 09.12.2020
(73) Patentinhaber: Olympus Winter & Ibe GmbH, 22045 Hamburg (DE)
(72) Erfinder: KIEDROWSKI, Gregor, 22397 Hamburg (DE)
(74) Vertreter: Hoener, Matthias

(56) Entgegenhaltungen:
- EP-A1- 2 617 348
- EP-A2- 1 776 918
- DE-A1-102016 111 363
- JP-A- S59 222 802

## Beschreibung

Die Erfindung betrifft ein medizinisches Endoskop der im Oberbegriff des Anspruchs 1 genannten Art, sowie ein Verfahren zu dessen Montage. Insbesondere betrifft die Erfindung medizinische Endoskope mit einem langgestreckten, semiflexiblen Schaftteil.

Medizinische Endoskope der erfindungsgemäßen Art haben ein langgestrecktes, semiflexibles Schaftteil und werden z. B. in der Urologie eingesetzt und dort als Urethroskope oder Ureteroskope bezeichnet. Urethroskope werden bei endoskopischen Arbeiten im Ureter und im Nierenbecken zur Beobachtung der Eingriffsstelle und zur Einführung von Durchgangsinstrumenten eingesetzt. Um endoskopisch, also unblutig zur Eingriffsstelle zu gelangen, wird das Urethroskop von außen durch die Urethra (Harnröhre) in die Blase vorgeschoben, von dort durch das Ostium (Mündung des Ureters mit Verschlussklappe) in den Ureter (Harnleiter) eingeführt und in diesem bis zum Operationsgebiet vorgeschoben, z. B. bis zu einem Stein oder einer Verengung im Ureter bzw. bis zum Nierenbecken. Um in diese Bereiche zu gelangen, weist das Schaftrohr des Urethroskops in der Regel eine Länge von mindestens etwa 330 mm auf.

Innerhalb des Schaftrohrs verlaufen in der Regel eine Optik zur Beobachtung des Operationsgebietes, mindestens ein Lichtleitfaserbündel zur Beleuchtung und ein oder mehrere Arbeitskanäle, durch die Durchgangsinstrumente, wie Katheter, Zangen, Steinfangkörper, Lithotriptoren (Steinzertrümmerungsgeräte) usw. an die Eingriffsstelle geführt werden können. Da der Ureter und insbesondere das Ostium sehr eng sind, ist der maximal tolerierbare Schaftdurchmesser stark begrenzt. Der Durchmesser sollte in der Regel nicht grösser als etwa 3 bis 4 mm sein. Daraus ergibt sich eine sehr lange und dünne Konfiguration des Schaftrohres. Die Optik kann vorteilhaft als Bildleiterfaserbündel ausgebildet sein. Dadurch werden Schaftrohre mit einer hinreichend großen Flexibilität ermöglicht (Semiflexibilität), die dazu beiträgt, dass Eingriffe besonders schonend durchgeführt werden können.

Die Hauptteile der Schaftrohre weisen in der Regel einen runden Querschnitt auf. Um den Durchmesser im distalen Endbereich auf ein Minimum zu reduzieren, wird dieser Bereich in einigen Endoskopen, wie den OES Pro Single Channel Ureteroscopen (Olympus) und den OES 4000 Dual Channel Ureteroscopen (Olympus), mit einem unrunden Querschnitt versehen. Je nach Anzahl der Arbeitskanäle ist der Endbereich in diesen Instrumenten dann entweder oval (ein Kanal) oder im Wesentlichen dreieckig (zwei Kanäle) ausgebildet. Der Umfang des distalen Endbereichs kann hierdurch reduziert werden, sodass dieser Endoskopabschnitt noch schonender zur Eingriffsstelle geführt werden kann. Derartige Ureteroskope sind beispielsweise in der DE 10 2016 111 363 A1 beschrieben.

Die Lichtleitfaserbündel können im Innenraum eines Endoskops frei eingezogen sein oder beispielsweise durch einen das Bündel umgebenden Schrumpfschlauch geschützt sein, d.h. einen Schlauch, der sich unter Hitzeeinwirkung um das Faserbündel herum zusammenzieht. Frei liegende Lichtleitfasern laufen bei einer elastischen Verformung des Schaftteils Gefahr, durch andere Komponenten des Schaftteils beschädigt zu werden, da diese Komponenten Druck oder Reibung auf das Lichtleitfaserbündel ausüben und so einzelne Fasern beschädigen können. Hierdurch kann die Lichtausbeute am distalen Ende der Faserbündel deutlich verringert werden. Ein umgebender Schrumpfschlauch schützt vor diesen Beschädigungen. In der vorstehendend genannten DE 10 2016 111 363 A1 wird alternativ eine flexible Verklebung der Lichtleitfasern im runden Schaftabschnitt vorgeschlagen. Diese Schutzeinrichtungen für Lichtleitfaserbündel wurden bislang nur im runden Abschnitt der Urethroskope verwendet, da es hier zu den stärksten Verformungen des Schaftes während eines Eingriffs kommt und das Beschädigungsrisiko am Größten ist.

Es hat sich nun herausgestellt, dass Beschädigungen der Lichtleitfasern nicht nur im runden, stärkeren Biegungen unterworfenen Schaftteil verursacht werden, sondern auch in dem in der Regel relativ wenig Biegungen unterworfenen, distalen Endbereich. In diesem Bereich ist aufgrund des deutlich reduzierten Durchmessers ein Schutz von Lichtleitfaserbündeln durch beispielsweise einen Schrumpfschlauch nicht möglich. Es besteht somit Bedarf an Endoskopen, deren Lichtleitfaserbündel bei der Verwendung nicht beschädigt werden und in denen gleichzeitig das vom Lichtleiter eingenommene Volumen im Innenraum des Schaftteils so gering wie möglich gehalten wird.

### Beschreibung der Erfindung

Gelöst wird die Aufgabe durch ein Endoskop, das die kennzeichnenden Merkmale des Anspruchs 1 aufweist, sowie durch Verfahren zu dessen Montage, welche die kennzeichnenden Merkmale des Anspruchs 13 aufweisen. Die Unteransprüche betreffen vorteilhafte Ausgestaltungen der Erfindung.

Erfindungsgemäß ist es insbesondere vorgesehen, die Lichtleitfasern eines Lichtleiters im distalen Endbereich des Endoskopschaftes mittels eines unflexiblen Klebstoffs zu verkleben. Dadurch wird der Lichtleiter im distalen Endbereich stabilisiert und vor Beschädigungen durch Verformung, Reibung oder Druck geschützt. Es ist insbesondere nicht erforderlich, den Lichtleiter in diesem Schaftabschnitt durch Ummantelung mit einem separaten Lichtleiterkanal, einem Schrumpfschlauch oder durch Verklebung mit einem flexiblen Klebstoff zu schützen. Es hat sich überraschenderweise herausgestellt, dass die Lichtleitfasern auch in diesem Bereich eines mechanischen Schutzes bedürfen. Darüber hinaus hat sich überraschenderweise herausgestellt, dass die schützende Wirkung mit einem unflexiblen Klebstoff erzielt werden kann. Es muss daher in dem distalen Endbereich nur eine einzige Art von Klebstoff verwendet werden, sodass es zu keinen Wechselwirkungen zwischen verschiedenen Klebstoffarten kommt.

Die Erfindung betrifft in einem ersten Aspekt ein medizinisches Endoskop mit einem langgestreckten Schaftteil mit einem distalen Endbereich mit unrundem Querschnitt und einem proximal davon angeordneten Schafthauptabschnitt, wobei das Schaftteil in Längsrichtung von mindestens einem, eine Vielzahl einzelner Fasern aufweisenden Lichtleiter durchlaufen wird, dadurch gekennzeichnet, dass Fasern des Lichtleiters im distalen Endbereich auf einer Länge von mindestens 50% des distalen Endbereichs mittels eines unflexiblen Klebstoffs verklebt sind, vorzugsweise auf der gesamten Länge des distalen Endbereichs.

Die erfindungsgemäßen medizinischen Endoskope weisen ein langgestrecktes Schaftteil mit einem distalen Endbereich mit unrundem Querschnitt und einem proximal davon angeordneten Schafthauptabschnitt sowie ein sich proximal daran anschließendes Bedienteil für den Operateur auf. Das Schaftteil ist bevorzugt durch ein abgedichtetes Gehäuse ausgebildet. Dieses kann zum Beispiel in Form eines Stielrohres ausgebildet sein. Mit anderen Worten umfasst das Schaftteil somit bevorzugt ein äußeres Stielrohr bzw. Außenrohr. Daneben kann das Schaftteil Innenrohre enthalten, die im Inneren des Außenrohres verlaufen, sowie diverse in das Schaftteil eingezogene Elemente, wie z.B. Arbeitskanäle, Faserrohre, Optikkanäle, weitere Lichtleiter und dergleichen.

Dem Fachmann sind geeignete Materialien, z. B. Stahl, bekannt, aus denen das Gehäuse des Schaftteils hergestellt sein kann. Insbesondere sind Materialien geeignet, die eine ausreichende Flexibilität des Schaftteils sicherstellen. So kann sich das Schaftteil bei Untersuchungen biegen, um den Organdurchlässen, insbesondere der Harnröhre und den Harnleitern zu folgen.

Es ist besonders bevorzugt, dass das medizinische Endoskop ein Urethroskop ist. Urethroskope (auch synonym: Ureteroskope) werden für Untersuchungen der Harnleiter eingesetzt und haben daher ein besonders langes Schaftteil, das eine entsprechende Flexibilität aufweisen muss. Alternativ kann das medizinische Endoskop aber auch jedes andere Endoskop sein, das einen semiflexiblen Schaftteil aufweist oder aufweisen kann. "Semiflexibel" bedeutet in diesem Zusammenhang, dass das Schaftteil im Einsatz begrenzt elastisch verformbar ist, d.h. sich biegen lässt und bei Nachlassen der biegenden Kraft in die ursprüngliche Form zurückkehrt.

Die Länge des Schaftteils ist so lang, dass eine bequeme Handhabung und die Erreichbarkeit der Operationsstelle gewährleistet ist. So kann das Schaftteil eine Länge von etwa 200 mm bis 600 mm, bevorzugt 300 mm bis 500 mm, stärker bevorzugt etwa 330 bis 430 mm aufweisen. Der Durchmesser des Schaftteils ist sehr gering. So kann das Schaftteil einen Durchmesser von etwa 0,5 mm bis 8 mm, vorzugsweise etwa 1 mm bis 5 mm, besonders bevorzugt 2 mm bis 4 mm aufweisen. Erfindungsgemäß ist das Verhältnis von Schaftdurchmesser zur Länge des Schaftes so gewählt, dass der Schaft mindestens abschnittsweise flexibel bzw. biegsam ist, insbesondere elastisch verformbar, d.h. reversibel biegsam. Es hat sich herausgestellt, dass diese geringe tolerierte Biegung die erfindungsgemäß im distalen Endbereich durch einen unflexiblen Klebstoff geschützten Lichtleitfasern nicht beschädigt.

Der distale Endbereich weist einen unrunden Querschnitt auf, das heißt der Querschnitt weicht in diesem Bereich von der Kreisform ab, d.h. ist nicht kreisförmig. Der Abschnitt kann z. B. oval oder dreieckig sein, wobei bei letzterer Alternative die Ecken der Dreiecksform abgerundet sind. Durch diese Querschnittsformen kann der Umfang des distalen Endbereichs auf ein Minimum reduziert werden, sodass der Anwender des Endoskops in der Lage ist, das distale Ende des Endoskops schonend in den gewünschten Bereich zu bewegen.

Im Gegensatz zu dem distalen Endbereich weist der Schafthauptabschnitt bevorzugt einen runden Querschnitt auf. Im Schafthauptabschnitt kann auch die Schaftwand dicker sein. Der Schafthauptabschnitt kann einen größeren Umfang aufweisen als der distale Endbereich. Es versteht sich, dass auch der maximale Durchmesser des Schafthauptabschnitts in diesen Ausführungsformen größer sein wird als der maximale Durchmesser des distalen Endbereichs. Auf diese Weise kann den größeren Belastungen Rechnung getragen werden, die auf den Schafthauptschnitt einwirken können.

Erfindungsgemäß erstreckt sich der "distale Endbereich" über den gesamten distalen Bereich, in dem der Schaftteil einen unrunden Querschnitt aufweist. Dies bedeutet, dass sich der distale Endbereich beispielsweise in der Regel nach proximal über den Bereich hinaus erstrecken wird, in dem das distale Linsensystem einer Optik angeordnet ist. Der distale Endbereich des Schaftteils kann dabei beispielsweise eine Länge von etwa 70 mm oder mehr aufweisen, bevorzugt von etwa 80 mm oder mehr, stärker bevorzugt von etwa 90 mm oder mehr, beispielsweise etwa 100 mm. Mit anderen Worten kann der distale Endbereich des Schaftteils eine Länge von etwa 70 mm bis 240 mm, bevorzugt von etwa 80 mm bis 170 mm, stärker bevorzugt von etwa 90 mm bis 150 mm aufweisen.

Das Schaftteil wird in Längsrichtung von mindestens einem, eine Vielzahl einzelner Fasern (Lichtleitfasern) aufweisenden Lichtleiter durchlaufen. Dies bedeutet, dass das Schaftteil von einem oder mehr Lichtleitern durchlaufen werden kann, beispielsweise ein, zwei oder drei Lichtleitern, wobei zwei Lichtleiter besonders bevorzugt sind. Folgende Beschreibung gilt für alle Lichtleiter, wenn mehrere in einem Schaftteil vorhanden sind.

Der eine Vielzahl einzelner Fasern aufweisende Lichtleiter erstreckt sich im Wesentlichen durch die gesamte Länge des Schaftteils, d. h. durch den Schaftteil von dem proximalen Ende des Schafthauptabschnitts bis zu dem distalen Ende des distalen Endbereichs. Da der Lichtleiter erfindungsgemäß im distalen Endbereich durch Verklebung mit einem unflexiblen Klebstoff geschützt ist, wird der Lichtleiter auch nach regelmäßigem Gebrauch des Endoskops im Wesentlichen intakt bleiben. Dies bedeutet z. B., dass auch nach mehrmaligem Gebrauch mehr als 70%, vorzugsweise mehr als 80%, stärker bevorzugt mehr als 90% der Fasern des Lichtleiters im distalen Endbereich des Schaftteils intakt sind. "Intakt" bedeutet in diesem Zusammenhang, dass die Lichtleitung durch die Faser nicht wesentlich durch Beschädigung, wie z. B. Zerreißen der Faser, beeinträchtigt wurde.

Der Lichtleiter ist geeignet, Licht einer Lichtquelle zum distalen Ende des Schaftteils zu leiten. Geeignete, eine Vielzahl einzelner Fasern aufweisende Lichtleiter sind dem Fachmann bekannt und werden routinemäßig in der Endoskopie bzw. Urethroskopie verwendet. Bevorzugt ist der Lichtleiter als Lichtleitfaserbündel ausgebildet, d.h. als ein Bündel aus Lichtleitfasern. Ein Lichtleitfaserbündel weist jeweils mehrere Lichtleitfasern auf, z.B. mehrere Glasfasern. Lichtleitfasern können z.B. Glasfasern oder auf Kunststoff basierende Fasern sein. Der Lichtleiter bzw. das Lichtleitfaserbündel ist an eine externe Lichtquelle koppelbar oder angeschlossen. Der Lichtleiter, d.h. das Lichtleitfaserbündel, hat vorzugsweise eine Dicke von 0,4 bis 0,8 mm, stärker bevorzugt von 0,5 bis 0,71 mm.

Der Begriff "Bündel" ist in diesem Zusammenhang nicht so zu verstehen, dass die Ansammlung von Lichtleitfasern zwingend einen runden Querschnitt aufweisen muss. Während der Lichtleiter, insbesondere das Lichtleitfaserbündel, einen runden Querschnitt aufweisen kann, sind andere Querschnittsformen, mittels derer sich der Lichtleiter unter Umständen besser in den freien Raum innerhalb des Schaftteils einpassen lässt, im Rahmen der Erfindung ebenso möglich. Für eine möglichst platzsparende Anordnung kann die Querschnittsform eines Lichtleitfaserbündels beispielsweise an die äußeren Konturen der im Inneren des Schaftteils verlaufenden weiteren Komponenten angepasst sein.

Die Fasern des Lichtleiters sind im distalen Endbereich mittels eines unflexiblen Klebstoffs verklebt. Durch die Verklebung werden die Lichtleitfasern innerhalb des distalen Endbereiches vor Druck und Reibung und somit vor Zerstörung während der Anwendung geschützt. Durch die Verklebung durchläuft der Lichtleiter den distalen Endbereich unmittelbar angrenzend an - bzw. lediglich durch eine Klebstoffschicht getrennt - weitere Kanälen, wie z. B. den Arbeitskanal und den Optikkanal (auch bekannt als Optikkanal). Ein zusätzlicher Schutz des Lichtleiters im distalen Endbereich mittels eines ummantelnden Schlauches oder Rohres ist nicht erforderlich.

Erfindungsgemäß sind Fasern des Lichtleiters im distalen Endbereich auf einer Länge von mindestens 50% des distalen Endbereichs mittels eines unflexiblen Klebstoffs verklebt, vorzugsweise von etwa 100%. Entsprechend sind Fasern des Lichtleiters vorzugsweise auf einer Länge (entlang der Längsachse des Lichtleiters bzw. Schaftteils) von 50% oder mehr, stärker bevorzugt auf einer Länge von 65% oder mehr, besonders bevorzugt auf 80% oder mehr der Länge des distalen Endbereichs mittels eines unflexiblen Klebstoffs verklebt. Anders gesagt sind Fasern des Lichtleiters vorzugsweise auf einer Länge von 50% bis 100%, stärker bevorzugt auf einer Länge von 65% bis 100%, am stärksten bevorzugt auf einer Länge von 80% bis 100% des distalen Endbereichs mittels eines unflexiblen Klebstoffs verklebt. Vorzugsweise erfolgt die Verklebung der Lichtleitfasern im Wesentlichen auf der gesamten Länge des distalen Endbereichs. Im Unterschied hierzu sind die Lichtleitfasern in üblichen Endoskopen lediglich an ihrem distalen Ende mittels eines unflexiblen Klebstoffs mit einer Linse oder dergleichen verklebt. Die übliche Verklebung erstreckt sich nicht über einen längeren Abschnitt des Schaftteils.

"Verklebt" bedeutet erfindungsgemäß, dass zwei oder mehr Fasern eines Lichtleitfaserbündels mittels eines Klebstoffs aneinander haften. Dabei kann es insbesondere ausreichend sein, die äußeren Fasern eines Lichtleitfaserbündels miteinander zu verkleben, um einen ausreichenden Schutz zu gewährleisten. In der Regel wird dabei durch Kapillarkräfte beim Auftragen des Klebstoffs auch Klebstoff in das Innere des Lichtleitfaserbündels gelangen. Es ist alternativ oder zusätzlich aber auch möglich, Fasern im Inneren eines Lichtleiters durch zusätzliche Einbringung von Klebstoff zwischen die Fasern miteinander zu verkleben.

Zusätzlich zu der Verklebung der Lichtleitfasern untereinander können eine oder mehrere Fasern auch mit anderen Komponenten des Endoskops verklebt sein. So kann der Lichtleiter beispielsweise innerhalb des distalen Endbereichs mittels des Klebstoffs mit der Schaftwandung verbunden sein, z. B. mit der Innenwand eines Außenrohres des Schaftteils. Hierdurch kann der mechanische Schutz der Lichtleitfasern noch mehr verbessert werden, da auf die Fasern einwirkende Reibungskräfte reduziert werden. Darüber hinaus kann auf diese Weise auch der Schaftinnenraum - insofern er nicht durch andere Durchgangsinstrumente und Innenrohre ausgefüllt ist - gegenüber dem Schaftaußenraum abgedichtet werden. In einer Ausführungsform dichtet der Klebstoff somit auch den distalen Endbereich gas- und/oder flüssigkeitsdicht ab. Dies bedeutet dass keine Gase und/oder Flüssigkeiten durch das distale Ende des Endoskops in das Schaftteilinnere eintreten können. Es versteht sich, dass eventuell im Inneren des Schaftteils verlaufende Kanäle, insbesondere Kanäle für Flüssigkeitsaustausch und Kanäle für Durchgangsinstrumente von dieser Abdichtungsfunktion des Klebstoffs ausgenommen sind. Der Klebstoff dichtet lediglich den freien Raum innerhalb des Schaftteils ab.

Der erfindungsgemäß zur Verklebung verwendete Klebstoff ist ein unflexibler Klebstoff. Dies bedeutet, dass sich der Klebstoff nach seinem Aushärten nur begrenzt mechanisch verformen lässt, insbesondere, dass der Klebstoff nach seinem Aushärten nicht oder nur unwesentlich elastisch verformbar bzw. biegbar ist. *Vice versa* bedeutet "biegsamer" somit erfindungsgemäß "leichter bzw. überhaupt elastisch verformbar". Es kann jeglicher unflexible Klebstoff zur Verklebung der Fasern des Lichtleiters im distalen Endbereich verwendet werden. Der Klebstoff sollte dabei nach seiner Aushärtung starr genug sein, um die Lichtleiter aneinander zu fixieren und vor wesentlicher Verformung zu schützen. Die folgenden Angaben beziehen sich jeweils auf die Eigenschaften des Klebstoffs nach seinem Aushärten.

Der Klebstoff ist schleifbar, d.h. er weist eine Härte auf, die ausreichend ist, um ihn schleifbar und polierbar zu machen. Geeignete unflexible, schleifbare Klebstoffe sind beispielsweise Klebstoffe mit einer Shore D-Härte von 60 oder mehr, vorzugsweise 70 oder mehr, stärker bevorzugt 75 oder mehr. Besonders geeignet sind z. B. Zweikomponentenklebstoffe. Bei diesen wird durch Vermischen zweier Klebstoffkomponenten eine chemische Aushärtungsreaktion gestartet. Zweikomponentenklebstoffe umfassen beispielsweise ungesättigte Polyesterharze, Epoxidharze, Methylmethacrylatklebstoffe und Fibrinklebstoffe. In einer besonders bevorzugten Ausführungsform ist der unflexible Klebstoff ein Epoxidklebstoff. Geeignete Epoxidklebstoffe sind im Handel erhältlich und dem Fachmann bekannt. Ein geeigneter Epoxidklebstoff ist z. B. EPO-TEK^{®} 354 (Epoxy Technology, Inc.).

An den distalen Endbereich schließt sich proximal ein Schafthauptabschnitt an. Der Schafthauptabschnitt kann unmittelbar an den distalen Endbereich angrenzen oder durch einen kurzen Übergangsabschnitt von dem distalen Endbereich getrennt sein. Der Übergangsabschnitt ist in der Regel nicht länger als 5 cm, d.h. 5 cm lang oder weniger, vorzugsweise 4 cm oder weniger, besonders bevorzugt 3 cm oder weniger. Der Schafthauptabschnitt kann aber auch unmittelbar an den distalen Endbereich angrenzen. Der Schafthauptabschnitt liegt somit zwischen dem distalen Endbereich und dem Bedienteil bzw. dem Hauptkörper des Endoskops, das zur Bedienung und zum Halten des Instrumentes dient. Bevorzugt grenzt unmittelbar proximal an den Schafthauptabschnitt das Bedienteil des Endoskops an.

Der Schafthauptabschnitt ist länger als der distale Endbereich und umfasst mehr als 50% der Länge des Schaftteils. Der Schafthauptabschnitt kann beispielsweise eine Länge von etwa 100 mm bis 500 mm, stärker bevorzugt 200 mm bis 380 mm, am stärksten bevorzugt 240 mm bis 350 mm aufweisen. Mit anderen Worten kann der Schafthauptabschnitt beispielsweise eine Länge von 100 mm oder mehr, vorzugsweise eine Länge von 200 mm oder mehr, stärker bevorzugt eine Länge von 300 mm oder mehr aufweisen. In einer bevorzugten Ausführungsform weist das Schaftteil einen distalen Endbereich mit einer Länge von etwa 80 bis 150 mm auf und einen proximal davon angeordneten Schafthauptabschnitt mit einer Länge von etwa 240 mm bis 380 mm auf. Der Schafthauptabschnitt wird bei der Benutzung des Instruments einem größeren Biegemoment ausgesetzt und verbiegt sich in der Regel stärker als der distale Endbereich. Mit anderen Worten ist insbesondere der Schafthauptabschnitt elastisch deformierbar. Hierdurch wird es dem Benutzer ermöglicht, während der Handhabung den Untersuchungsort durch die Organdurchlässe zu erreichen, ohne Organe zu beschädigen. Die bei der Handhabung entstehende Biegung im Schaftteil wird in der Regel, d.h. bei sachgerechter, normaler Benutzung des Endoskops (passiv) reversibel sein, so dass die Deformation bei Entfernung der Belastung, z. B. bei Entfernen des Endoskops aus dem Körper des Patienten, verschwindet, so dass das Schaftteil wieder seine ursprüngliche Form annimmt. Vorzugsweise weist der Schafthauptabschnitt auf mindestens 70% seiner Länge, vorzugsweise mindestens 80% seiner Länge einen im Wesentlichen runden Querschnitt auf.

Da der Durchmesser und der Umfang des Schaftteils im Schafthauptabschnitt größer sein können als der Durchmesser bzw. der Umfang im distalen Endbereich, können die Lichtleitfasern hier durch verschiedene Mittel geschützt werden. So kann der Lichtleiter im Schafthauptabschnitt beispielsweise durch einen Schlauch ummantelt oder ebenfalls verklebt sein, wobei es bevorzugt ist, dass diese Verklebung mit einem flexiblen Klebstoff, wie einem Silikonklebstoff, erfolgt. Alternativ und besonders bevorzugt ist allerdings der Schutz des Lichtleiters im Schafthauptabschnitt mittels eines den Lichtleiter umgebenden Schlauchs. Bevorzugt durchläuft der Lichtleiter im Schafthauptabschnitt daher mindestens abschnittsweise einen Schlauch, vorzugsweise einen Schrumpfschlauch. Dieser Schlauch kann beispielsweise ein Silikonschlauch sein. Durch die Verwendung eines Schlauches wird sichergestellt, dass sich bei einer Biegung eines flexiblen Abschnitts des Schaftteils der Lichtleiter der Bewegung des Schaftteils anpassen kann und gleichzeitig vor Druck und Reibung durch andere im Schaftteil enthaltene Komponenten (z.B. Kanäle) geschützt ist. Gegenüber einer Verklebung mit einem flexiblen Klebstoff hat die Verwendung eines Schlauches den Vorteil, dass in der Montage des Instruments nicht unterschiedliche, potentiell miteinander wechselwirkende Klebstoffe verwendet werden müssen.

In einem weiteren Aspekt betrifft die Erfindung ein Verfahren zur Montage eines hierin beschriebenen, erfindungsgemäßen Endoskops, dadurch gekennzeichnet, dass es Schritte umfasst, bei denen man (a) einen unflexiblen Klebstoff innerhalb eines distalen Endbereichs eines Schaftteils des Endoskops an mindestens einer Position platziert, (b) einen, eine Vielzahl einzelner Fasern aufweisenden, Lichtleiter in das Schaftteil einbringt, und (c) den Klebstoff erhärtet oder aushärten lässt.

Im Schritt (a) wird der noch nicht ausgehärtete Klebstoff - beispielsweise ein Zweikomponentenklebstoff - unmittelbar nach dem Vermischen beider Klebstoffkomponenten - innerhalb des distalen Endbereichs an mindestens einer Position platziert. Dabei kann beispielsweise mit Hilfe einer Kanüle an der Innenwandung des Außenrohres eines Schaftteiles eine Portion des Klebstoffs platziert werden. An einer oder mehreren weiteren Positionen, die vorzugsweise jeweils in Längsrichtung voneinander beabstandet sind, des Außenrohres werden dann weitere Portionen des Klebstoffs platziert.

Unmittelbar im Anschluss an das Platzieren des Klebstoffs, insbesondere vor dem Aushärten des Klebstoffs, wird der Lichtleiter in das Schaftteil, insbesondere in den mit Klebstoffportionen ausgestatteten distalen Endbereich des Schaftteils eingebracht. Weitere Komponenten, wie Faser- und Arbeitskanäle können zeitgleich oder zeitnah ebenfalls eingebracht werden, um eine dichte Verklebung zu gewährleisten. Der Lichtleiter kann beispielsweise in das Schaftteil eingezogen oder eingeschoben werden, wobei das Einziehen in das Schaftteil bevorzugt ist.

Nach dem Einbringen des Klebstoffs und des Lichtleiters und potentieller weiterer Komponenten in den Schaftteil wird der Klebstoff erhärtet oder aushärten gelassen. Dem Fachmann sind geeignete Verfahren, die sich je nach Art des verwendeten unflexiblen Klebstoffs unterscheiden mögen, bekannt.

### Kurze Beschreibung der Figuren

In den Zeichnungen sind Ausführungsbeispiele der Erfindung schematisch dargestellt. Es zeigen:
- Fig. 1: eine schematische Seitenansicht eines erfindungsgemäßen Urethroskops,
- Fig. 2: einen Schnitt nach Linie III-III in Fig. 1 durch den Schafthauptabschnitt des Schaftteils,
- Fig. 3a: einen Schnitt nach Linie IV-IV in Fig. 1 durch einen distalen Endbereich des Schaftteils, wobei das Schaftteil einen Arbeitskanal und einen Optikkanal und der Endbereich einen unrunden, ovalen Querschnitt aufweist, und
- Fig. 3b: einen Schnitt nach Linie IV-IV in Fig. 1 durch einen distalen Endbereich eines alternativen Schaftteils, wobei das Schaftteil zwei Arbeitskanäle und einen Optikkanal und der Endbereich einen unrunden, im Wesentlichen dreieckigen Querschnitt aufweist.

### Ausführungsbeispiele

Weitere Vorteile, Kennzeichen und Merkmale der vorliegenden Erfindung werden bei der nachfolgenden detaillierten Beschreibung von Ausführungsbeispielen anhand der beigefügten Zeichnungen deutlich. Allerdings ist die Erfindung nicht auf diese Ausführungsbeispiele beschränkt.

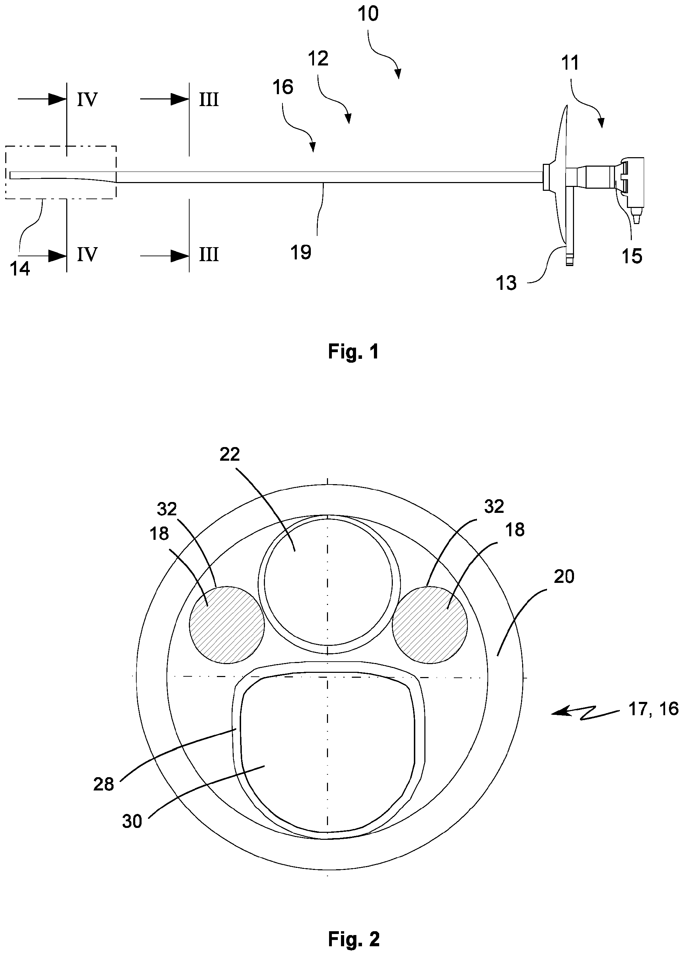

Fig. 1 zeigt eine Seitenansicht eines erfindungsgemäßen Endoskops 10 mit einem Hauptkörper 11 und einem sich daran distal anschließenden lang erstreckten rohrförmigen, in den Patientenkörper einzuführenden Schaftteil 12. Der Hauptkörper 11 kann z. B. ein Okular und ein Einführungsteil umfassen. Das Schaftteil 12 und der Hauptkörper 11 sind in nicht näher erläuterter Weise abgedichtet miteinander verbunden, beispielsweise verklebt, verlötet oder dergleichen.

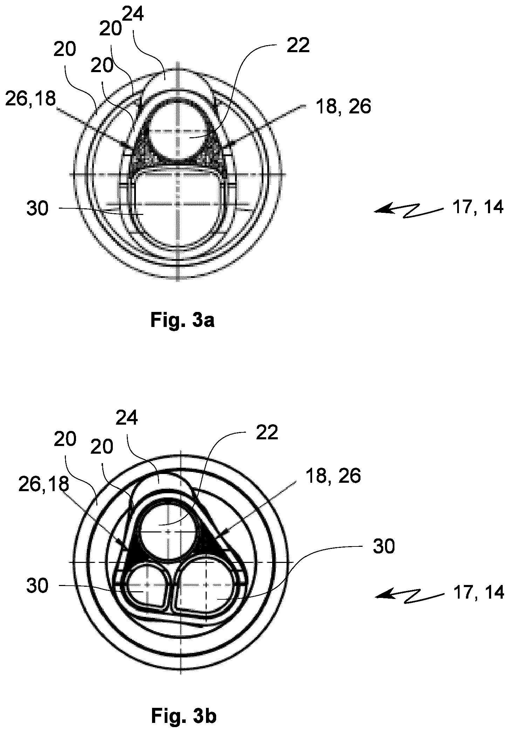

In einem zum Anschluss eines nicht dargestellten, externen Lichtleiters dienenden Anschlussstutzen 13 am Hauptkörper 11 kann das proximale Ende eines in Figs. 2 und 3 dargestellten Lichtleiters 18 in Form eines Lichtleitfaserbündels befestigt werden, der den Innenraum des Schaftteils 12 in Längsrichtung bis zum distalen Ende des Schaftteils 12 durchläuft.

Ferner kann vom Hauptkörper 11 ein Okularstutzen 15 abgehen, durch den eine Optik verläuft, die durch den Schaftteil 12 ebenfalls bis zum distalen Ende des Endoskops 10 geführt ist. Am proximalen Ende der Optik kann am Okularstutzen 15 ein Okular vorgesehen sein.

Die Länge des Schaftteiles 12 beträgt bei einem Endoskop 10 üblicher Ausbildung, das, wie das dargestellte, als Urethroskop ausgebildet ist, z. B. 400 bis 500 mm. Der Außendurchmesser des Schaftteiles 12, insbesondere der Außendurchmesser des Außenrohrs 19 des Schaftteils 12, soll maximal etwa 4 mm nicht überschreiten. Bei einem solchen Durchmesser des Außenrohrs 19 ergibt sich, im Wesentlichen unabhängig von der Wandstärke, eine gewisse Formsteifigkeit des Rohres, die bei Biegung des Rohres zu einer Längung des Materiales auf der Außenseite und einer Stauchung des Materials auf der Innenseite (bezogen auf den Mittelpunkt des Biegeradius) führt.

In dem Schaftteil 12 des in Fig. 1 dargestellten Endoskops 10 ist der distale Endbereich 14 mit einem geringeren Außendurchmesser und Umfang ausgebildet, als der proximal davon angeordnete Schafthauptabschnitt 16. Dadurch lässt sich der distale Endbereich 14 besonders schonend zur Eingriffsstelle führen. Der distale Endbereich 14 weist eine Länge von etwa 10 cm auf.

An seinem distalen Ende ist das Schaftteil 12 bzw. der distale Endbereich 14 auf geeignete Weise abgedichtet verschlossen, z. B. mit einem Fenster für eine Optik im in Figs. 2 und 3 dargestellten Optikkanal 22 und mit einer ausreichend dichten Verklebung des oder der in Figs. 2 und 3 dargestellten Lichtleiters 18. Die Verklebung am distalen Ende erfolgt in üblicher Ausführung beispielsweise durch einen unflexiblen Klebstoff. Das gut abgedichtete Schaftteil 12 sorgt dafür, dass die im Inneren angeordnete Optik und der oder die Lichtleiter 18 nicht durch beim Arbeiten mit dem Endoskop 10 oder bei der Sterilisation eindringendes Wasser oder Dampf beeinträchtigt werden. Darüber hinaus ist die unflexible Verklebung in dem distalen Endbereich 14 auf erfindungsgemäße Weise über die gesamte Länge des distalen Endbereichs 14 fortgesetzt. Dies dient einerseits zum Schutz des oder der in Fig. 2 und 3 dargestellten Lichtleiters 18 und andererseits zum noch besseren gas- und flüssigkeitsdichten Abdichten des Schaftteils 12.

Die Querschnittsdarstellung 17 der Fig. 2 zeigt, dass der Schaftteil 12 in einem Schafthauptabschnitt 16 eine den Außenumfang bestimmende Schaftwandung 20 runden Querschnitts aufweist, die vorzugsweise als ein Metallrohr ausgebildet sein kann. Der Schafthauptabschnitt 16 sowie der distale Endbereich 14 sind elastisch verformbar, d.h. reversibel biegsam, und können sich daher während des operativen Eingriffs flexibel verformen.

Der von dieser Schaftwandung 20 umschlossene Innenraum des Schaftteiles 12 nimmt in dem dargestellten Ausführungsbeispiel zwei innere Kanäle auf, den Optikkanal 22 und den Arbeitskanal 30. Sowohl der Optikkanal 22 als auch der Arbeitskanal 30 können als Metallrohre ausgebildet sein, zum Beispiel mit entsprechenden Wandungen 28 aus Metall, z. B. Stahl. Der Optikkanal 22 umschließt die Optik, die das betrachtete Bild von einem am distalen Ende des Schaftteiles 12 angeordneten Objektiv zum Okular überträgt. Der Arbeitskanal 30 ist als freier Kanal ausgebildet, in dem ein Instrument (z. B. ein Laserlithotriptor) durch einen nicht dargestellten Anschlussstutzen hindurch vorgeschoben werden kann, dessen proximal vorgesehener Lasergenerator ebenfalls nicht dargestellt ist.

Im Innenraum des Schaftteils 12 sind ferner zwei Lichtleiter 18 verlegt, die vorzugsweise als Lichtleitfaserbündel ausgebildet sind, und zur Beleuchtung des Betrachtungsfeldes dienen. Die Lichtleiter 18 sind in der dargestellten Ausführungsform mit einem flexiblen Schrumpfschlauch 32 aus Silikon ummantelt, der die Lichtleiter 18 vor Beschädigung durch Druck und Reibung schützt. Alternativ ist es auch möglich, die Lichtleiter 18 jeweils durch Verklebung mit einem flexiblen Klebstoff, wie einem Silikonklebstoff,- zu schützen. Bei einer solchen Verklebung sind jedoch bei der Instrumentenmontage die möglichen Wechselwirkungen zwischen verschiedenen Klebstoffarten zu berücksichtigen.

Am proximalen Ende des Schaftteils 12 kann der Lichtleiter 18 über einen Lichtleiteranschluss aus dem Hauptkörper 11 herausgeführt sein. Dort kann ein Lichtleiterverbindungskabel an einer nicht dargestellten Kaltlichtquelle angeschlossen werden.

Fig. 3a und Fig. 3b zeigen jeweils einen Querschnitt 17 durch einen distalen, unrunden Endbereich 14 verschiedener Schaftteile 12.

Es ist erkennbar, dass das in Fig. 3a dargestellte Schaftteil 12 in einem distalen Endbereich 14 einen im Wesentlichen ovalen Außenquerschnitt aufweist, während es - wie im Hintergrund von Fig. 3a erkennbar - proximal von diesem Endbereich 14 einen Schafthauptabschnitt 16 mit einem runden Außenquerschnitt aufweist. Das in Fig. 3a gezeigte Schaftteil 12 ist Teil eines 1-Kanal-Instruments, da das Schaftteil 12 erkennbar einen einzigen Arbeitskanal 30 aufweist.

Es ist ferner erkennbar, dass das in Fig. 3b dargestellte Schaftteil 12 in seinem distalen Endbereich 14 einen im Wesentlichen dreieckigen Außenquerschnitt mit abgerundeten Ecken aufweist, während es - wie im Hintergrund von Fig. 3b erkennbar - proximal von diesem Endbereich 14 einen Schafthauptabschnitt 16 mit einem runden Außenquerschnitt aufweist. Das in Fig. 3b gezeigte Schaftteil 12 ist Teil eines 2-Kanal-Instruments, da das Schaftteil erkennbar zwei Arbeitskanäle 30 aufweist.

Durch den ovalen bzw. dreieckigen Außenquerschnitt der distalen Endbereiche 14, weist der jeweils gezeigte distale Endbereich 14 des Schaftteils 12 einen gegenüber dem Schafthauptabschnitt 16 deutlich reduzierten Umfang auf.

Neben dem oder den Arbeitskanälen 30 weisen die Schaftteile 12 einen Optikkanal 22 auf, in den eine Optik eingezogen sein kann. Auf dem Schaftteil 12 ist ferner eine Dilatationsnase 24 angeordnet. Es ist erkennbar, dass die Lichtleiter 18 im Wesentlichen den gesamten freien Raum innerhalb des Schaftteils 12 ausfüllen. Die Lichtleiter 18 sind dabei zum Schutz gegen Brechen und Schutz vor Reibung mit einem unflexiblen EpoxidKlebstoff verklebt. Zur Verklebung wurden an mehreren Positionen des jeweiligen Endbereichs 14 an der Innenseite der Schaftwandung 20 Portionen des Klebstoffs platziert und anschließend Optikkanal 22, Arbeitskanal 30 oder Arbeitskanäle 30 und die Lichtleiter 18 in das Außenrohr 19 eingebracht. Dann wurde der Epoxidklebstoff aushärten gelassen. Die Verklebung erfolgte über die gesamte Länge des distalen Endbereichs 14.

Obwohl die vorliegende Erfindung anhand der Ausführungsbeispiele detailliert beschrieben worden ist, ist für den Fachmann selbstverständlich, dass die Erfindung nicht auf diese Ausführungsbeispiele beschränkt ist, sondern dass vielmehr Abwandlungen in der Weise möglich sind, dass einzelne Merkmale weggelassen oder andersartige Kombinationen der vorgestellten Einzelmerkmale verwirklicht werden können, sofern der Schutzbereich der beigefügten Ansprüche nicht verlassen wird. Die vorliegende Offenbarung schließt sämtliche Kombinationen der vorgestellten Einzelmerkmale ein.

### Bezugszeichenliste

- 10: Endoskop
- 11: Hauptkörper
- 12: Schaftteil
- 13: Anschlussstutzen
- 14: distaler Endbereich
- 15: Okularstutzen
- 16: Schafthauptabschnitt
- 17: Querschnitt Schaftteil
- 18: Lichtleiter
- 19: Außenrohr
- 20: Schaftwandung
- 22: Optikkanal
- 24: Dilatationsnase
- 26: unflexibler Klebstoff
- 28: Wandung des Arbeitskanals
- 30: Arbeitskanal
- 32: Schrumpfschlauch

## Patentansprüche

1. Medizinisches Endoskop (10) mit einem langgestreckten Schaftteil (12) mit einem distalen Endbereich (14) mit einem unrunden, nicht kreisförmigen Querschnitt und einem proximal davon angeordneten Schafthauptabschnitt (16) mit einem runden Querschnitt, wobei das Schaftteil (12) in Längsrichtung von mindestens einem, eine Vielzahl einzelner Fasern aufweisenden Lichtleiter (18) durchlaufen wird, **dadurch gekennzeichnet, dass** Fasern des Lichtleiters im distalen Endbereich (14) auf einer Länge von mindestens 50% des distalen Endbereichs (14) mittels eines nicht elastisch verformbaren Klebstoffs verklebt sind.

2. Medizinisches Endoskop (10) nach Anspruch 1, **dadurch gekennzeichnet, dass** der distale Endbereich (14) eine Länge von 40 mm oder mehr aufweist.

3. Medizinisches Endoskop (10) nach einem der vorangehenden Ansprüche, **dadurch gekennzeichnet, dass** Fasern des Lichtleiters (18) im distalen Endbereich (14) auf einer Länge von mindestens 65% des distalen Endbereichs (14) mittels eines nicht elastisch verformbaren Klebstoffs verklebt sind.

4. Medizinisches Endoskop (10) nach einem der vorangehenden Ansprüche, **dadurch gekennzeichnet, dass** der Schafthauptabschnitt (16) einen größeren Umfang aufweist als der distale Endbereich (14).

5. Medizinisches Endoskop (10) nach einem der vorangehenden Ansprüche, **dadurch gekennzeichnet, dass** der Lichtleiter (18) im Schafthauptabschnitt (16) mindestens abschnittsweise einen Schlauch durchläuft.

6. Medizinisches Endoskop (10) nach einem der vorangehenden Ansprüche, **dadurch gekennzeichnet, dass** der Klebstoff schleifbar ist.

7. Medizinisches Endoskop (10) nach einem der vorangehenden Ansprüche, **dadurch gekennzeichnet, dass** der Klebstoff eine Shore D-Härte von 70 oder mehr aufweist.

8. Medizinisches Endoskop (10) nach einem der vorangehenden Ansprüche, **dadurch gekennzeichnet, dass** der Klebstoff ein Zweikomponentenklebstoff ist.

9. Medizinisches Endoskop (10) nach einem der vorangehenden Ansprüche, **dadurch gekennzeichnet, dass** der Klebstoff ein Epoxidklebstoff ist.

10. Medizinisches Endoskop (10) nach einem der vorangehenden Ansprüche, **dadurch gekennzeichnet, dass** der Lichtleiter (18) innerhalb des distalen Endbereichs (14) mittels des Klebstoffs mit der Schaftwandung (20) verbunden ist.

11. Medizinisches Endoskop (10) nach einem der vorangehenden Ansprüche, **dadurch gekennzeichnet, dass** der Klebstoff den distalen Endbereich (14) gas- und/oder flüssigkeitsdicht abdichtet.

12. Medizinisches Endoskop (10) nach einem der vorangehenden Ansprüche, **dadurch gekennzeichnet, dass** der distale Endbereich (14) einen geringeren Durchmesser als der Schafthauptabschnitt (16) aufweist.

13. Verfahren zur Montage eines medizinischen Endoskops (10) gemäß einem der vorangehenden Ansprüche, **dadurch gekennzeichnet, dass** es Schritte umfasst, bei denen man
(a) einen nicht elastisch verformbaren Klebstoff innerhalb eines distalen Endbereichs (14) eines Schaftteils (12) des Endoskops (10) an mindestens einer Position platziert,
(b) einen, eine Vielzahl einzelner Fasern aufweisenden, Lichtleiter (18) in das Schaftteil einbringt, und
(c) den Klebstoff erhärtet oder aushärten lässt.

## Claims

1. Medical endoscope (10) having an elongate shaft part (12) with a distal end region (14) having a non-round, non-circular cross section and a shaft main portion (16), which is arranged in the proximal direction from said distal end region and has a round cross section, wherein at least one light guide (18) comprising a multiplicity of individual fibres passes through the shaft part (12) in a longitudinal direction, **characterized in that** fibres of the light guide in the distal end region (14) are adhesively bonded by means of a non-elastically deformable adhesive over at least 50% of the length of the distal end region (14).

2. Medical endoscope (10) according to Claim 1, **characterized in that** the distal end region (14) has a length of 40 mm or more.

3. Medical endoscope (10) according to either of the preceding claims, **characterized in that** fibres of the light guide (18) in the distal end region (14) are adhesively bonded by means of a non-elastically deformable adhesive over at least 65% of the length of the distal end region (14).

4. Medical endoscope (10) according to one of the preceding claims, **characterized in that** the shaft main portion (16) has a larger circumference than the distal end region (14).

5. Medical endoscope (10) according to one of the preceding claims, **characterized in that** the light guide (18) in the shaft main portion (16) runs through a hose at least in certain portions.

6. Medical endoscope (10) according to one of the preceding claims, **characterized in that** the adhesive is sandable.

7. Medical endoscope (10) according to one of the preceding claims, **characterized in that** the adhesive has a Shore D hardness of 70 or more.

8. Medical endoscope (10) according to one of the preceding claims, **characterized in that** the adhesive is a two-component adhesive.

9. Medical endoscope (10) according to one of the preceding claims, **characterized in that** the adhesive is an epoxy adhesive.

10. Medical endoscope (10) according to one of the preceding claims, **characterized in that** the light guide (18) within the distal end region (14) is connected to the shaft wall (20) by means of the adhesive.

11. Medical endoscope (10) according to one of the preceding claims, **characterized in that** the adhesive seals off the distal end region (14) in a gas-tight and/or fluid-tight manner.

12. Medical endoscope (10) according to one of the preceding claims, **characterized in that** the distal end region (14) has a smaller diameter than the shaft main portion (16).

13. Method for assembling a medical endoscope (10) according to one of the preceding claims, **characterized in that** it comprises the following steps:
(a) positioning a non-elastically deformable adhesive at least at one position within a distal end region (14) of a shaft part (12) of the endoscope (10),
(b) introducing a light guide (18) comprising a multiplicity of individual fibres into the shaft part, and
(c) allowing the adhesive to harden or cure.

## Revendications

1. Endoscope médical (10) avec une partie de tige allongée (12) avec une zone d'extrémité distale (14) ayant une section transversale non ronde, non circulaire et une section principale de tige (16) agencée de manière proximale par rapport à celle-ci, ayant une section transversale ronde, la partie de tige (12) étant traversée dans la direction longitudinale par au moins un guide de lumière (18) présentant une pluralité de fibres individuelles, **caractérisé en ce que** des fibres du guide de lumière sont collées dans la zone d'extrémité distale (14) sur une longueur d'au moins 50 % de la zone d'extrémité distale (14) au moyen d'un adhésif non déformable élastiquement.

2. Endoscope médical (10) selon la revendication 1, **caractérisé en ce que** la zone d'extrémité distale (14) présente une longueur de 40 mm ou plus.

3. Endoscope médical (10) selon l'une quelconque des revendications précédentes, **caractérisé en ce que** des fibres du guide de lumière (18) sont collées dans la zone d'extrémité distale (14) sur une longueur d'au moins 65 % de la zone d'extrémité distale (14) au moyen d'un adhésif non déformable élastiquement.

4. Endoscope médical (10) selon l'une quelconque des revendications précédentes, **caractérisé en ce que** la section principale de tige (16) présente une circonférence plus grande que la zone d'extrémité distale (14) .

5. Endoscope médical (10) selon l'une quelconque des revendications précédentes, **caractérisé en ce que** le guide de lumière (18) traverse au moins par sections un tube dans la section principale de tige (16).

6. Endoscope médical (10) selon l'une quelconque des revendications précédentes, **caractérisé en ce que** l'adhésif est abrasable.

7. Endoscope médical (10) selon l'une quelconque des revendications précédentes, **caractérisé en ce que** l'adhésif présente une dureté Shore D de 70 ou plus.

8. Endoscope médical (10) selon l'une quelconque des revendications précédentes, **caractérisé en ce que** l'adhésif est un adhésif à deux composants.

9. Endoscope médical (10) selon l'une quelconque des revendications précédentes, **caractérisé en ce que** l'adhésif est un adhésif époxy.

10. Endoscope médical (10) selon l'une quelconque des revendications précédentes, **caractérisé en ce que** le guide de lumière (18) est relié à la paroi de la tige (20) à l'intérieur de la zone d'extrémité distale (14) au moyen de l'adhésif.

11. Endoscope médical (10) selon l'une quelconque des revendications précédentes, **caractérisé en ce que** l'adhésif rend la zone d'extrémité distale (14) étanche aux gaz et/ou aux liquides.

12. Endoscope médical (10) selon l'une quelconque des revendications précédentes, **caractérisé en ce que** la zone d'extrémité distale (14) présente un diamètre inférieur à celui de la section principale de tige (16).

13. Procédé d'assemblage d'un endoscope médical (10) selon l'une quelconque des revendications précédentes, **caractérisé en ce qu'**il comprend des étapes selon lesquelles
(a) un adhésif non déformable élastiquement est placé à l'intérieur d'une zone d'extrémité distale (14) d'une partie de tige (12) de l'endoscope (10) en au moins une position,
(b) un guide de lumière (18) présentant une pluralité de fibres individuelles est introduit dans la partie de tige, et
(c) l'adhésif est durci ou laissé durcir.
